# EUROPEAN PATENT APPLICATION

(11) **EP 4 032 462 A1**
(43) Date of publication of application: **27.07.2022**
(21) Application number: 19945999.1
(22) Date of filing: 31.10.2019
(51) Int. Cl.: A61B 3/135, A61B 3/00, H04M 1/21, H04M 1/725

(54) **PORTABLE SLIT LAMP DEVICE COUPLABLE TO IMAGE CAPTURE DEVICE**

(30) Priority: 17.09.2019 KR 20190114128
(71) Applicant: Welsmeditech Co., Ltd., Cheonan-si, Chungcheongnam-do 31035 (KR)
(72) Inventor: HWANG, Kyong Won, Seoul 01197 (KR); MOON, Pyoungsu, Cheonan-si, Chungcheongnam-do 31101 (KR)
(74) Representative: Gulde & Partner
(86) International application number: PCT/KR2019/014622
(87) International publication number: WO 2021/054516

(57) **Abstract**

The present invention relates to a portable slit lamp device which can be coupled to an image capture device such as a smartphone.

## Description

### [Technical Field]

The present invention relates to a portable slit lamp device capable of being coupled to an image capture device such as a smartphone.

### [Background Art]

In the field of ophthalmology, slit lamps are used to observe optical cut surfaces of eyes. When slit lamp visible light passes through a slit and has a shape extending vertically to generate a slit lamp visible light ray, the slit lamp visible light ray can be obliquely emitted to an examinee's eye to observe a cut surface.

FIG. 1 is an image of an examinee's eye to which a slit lamp light is emitted.

FIG. 2 (a) is a schematic top view of the examinee's eye, and FIG. 2 (b) is a schematic side view of the examinee's eye.

Conventionally, although complex and heavy slit lamp cameras such as microscopes are used, due to the development of optical technology and portable cameras or smartphone cameras, slit lamp devices to be coupled to the cameras have also been developed.

Related arts will be reviewed.

Total 188 patents related to a slit lamp, of which slit lamp light is obliquely emitted to an eye and portability is excellent, have been checked around the world (as of May 2019), and typical 13 patents have been reviewed among the 188 patents in the present invention, and the following common problems have been checked.

First, there are several patents related to a slit lamp device that is attached to an image capture device such as a smartphone, uses the same light source as a rear camera of a smartphone and a flashlight, and does not consider a different camera position for each type of smartphones, a distance between a camera and a light source, and the like, and thus types of smartphones for which slit lamp cameras can be used are very limited. That is, the patents have low versatility.

Second, a slit lamp visible light ray should be obliquely emitted to an examinee's eye, but there is no function of adjusting an emission angle or, even when there is a function of adjusting an emission angle, a very complex mechanical structure is employed. When the mechanical structure is complex, a slit lamp device becomes heavy and portability thereof decreases.

Third, a slit lamp visible light ray should be emitted from both right and left sides with respect to an examinee's eye, but there are few patents having such a function. In this case, although a user can turn a slit lamp camera and a smartphone coupled to the slit lamp camera upside down and use the slit lamp camera and the smartphone, there is a problem in that it is difficult to capture an image when the smartphone is held upside down.

Fourth, in some patents, although light emitted from a light source with a different wavelength from a visible light source is further used, in this case, an optical path becomes excessively complex, and thus portability decreases. Particularly, since visible light and light with a different wavelength from visible light are emitted as the same light, effectiveness is questionable.

### (Patent Document)

(Patent Document 1) KR 10-1922609
(Patent Document 2) JP 6118477
(Patent Document 3) KR 10-1037460
(Patent Document 4) JP 3197418 U
(Patent Document 5) JP 5285994
(Patent Document 6) JP 4495292
(Patent Document 7) JP 4094378
(Patent Document 8) CN 106859589 B
(Patent Document 9) CN 107280632 A
(Patent Document 10) CN 104207751 B
(Patent Document 11) KR 10-1396044
(Patent Document 12) KR 10-1817037
(Patent Document 13) KR 10-1591133

### [Technical Problem]

The present invention is directed to providing a slit lamp device capable of being applied to any smartphone, emitting light from both left and right sides with respect to an examinee's eye, controlling an emission angle, emitting light with a different wavelength from visible light in addition to the visible light, and having superior portability.

### [Technical Solution]

One aspect of the present invention provides a portable slit lamp device (100) which is installed on an image capture device (200) and used, the portable slit lamp device (100) including a housing (110) provided with a plurality of mirrors, an eye contact part (120) provided on the housing (110) toward an examinee's eye, and a coupling part (130) provided on the housing (110) toward the image capture device (200), wherein the portable slit lamp device (100) and the image capture device (200) are coupled by the coupling part (130) so that a central axis of the eye contact part (120) is collinear with an axis of a camera (C) of the image capture device (200), a visible light source (140) is positioned in the housing (110), and by manipulating a mirror (M2), visible light emitted by the visible light source (140) is totally reflected by the mirror (M2), is reflected by a right reflecting mirror (M2'), and is emitted from a right side of an examinee's eye as slit visible light, or the visible light emitted by the visible light source (140) passes the mirror (M2), is reflected by a left reflecting mirror (M4'), and is emitted from a left side of the examinee's eye as slit visible light.

An additional light source (150), which emits light with a different wavelength from visible light, may be positioned in the housing (110), and the mirror (M2) may totally reflect the visible light and allow the additional light to pass through the mirror (M2).

The visible light source (140) and the additional light source (150) may be provided on different surfaces in the housing (110), a mirror (M1) may be positioned at a portion at which a visible light ray emitted by the visible light source (140) and an additional light ray emitted by the additional light source (150) come into contact with each other, the mirror (M1) totally may reflect the visible light and allow the additional light to pass through the mirror (M1), and the visible light ray and the additional light ray may be the same at downstream from the mirror (M1).

The mirror (M2) may be positioned on a path of the same ray at downstream from the mirror (M1), a mirror (M3) may be positioned at downstream from the mirror (M2), the mirror (M3) may allow the visible light to pass through the mirror (M3) and totally reflect the additional light, and the mirror (M3) may be positioned on a line extending from the central axis of the eye contact part (120) that is collinear with the axis of the camera (C) of the image capture device (200).

The right reflecting mirror (M2') may be positioned on a path of the visible light ray totally reflected by the mirror (M2), a mirror (M4) may be positioned on a path of the visible light ray passing through the mirror (M3), the mirror (M4) may totally reflect the visible light, and the left reflecting mirror (M4') may be positioned on a path of the totally reflected visible light ray.

The right reflecting mirror (M2') and the left reflecting mirror (M4') may be rotatable.

### [Advantageous Effects]

The present invention can provide a slit lamp device capable of being applied to any smartphone, emitting light from both left and right sides with respect to an examinee's eye, controlling an emission angle, emitting light with a different wavelength from visible light in addition to the visible light, and having superior portability.

### [Description of Drawings]

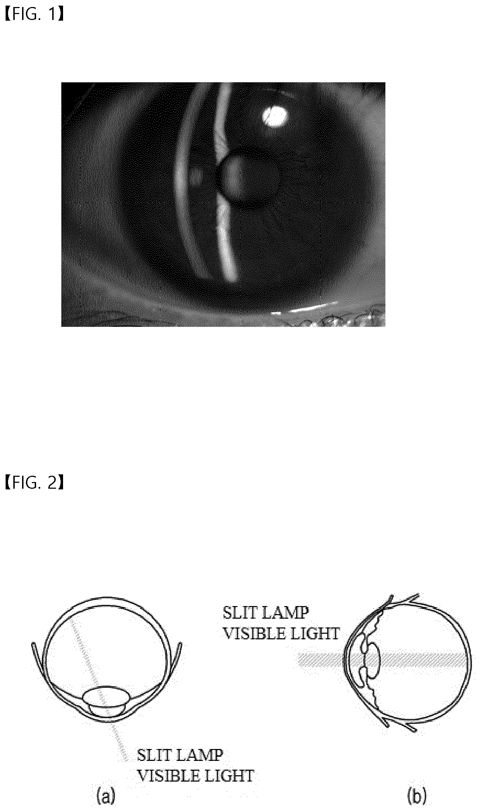

FIG. 1 is an image of an examinee's eye captured using a slit lamp.
FIG. 2 is a set of schematic views for describing an image capturing method using a slit lamp.

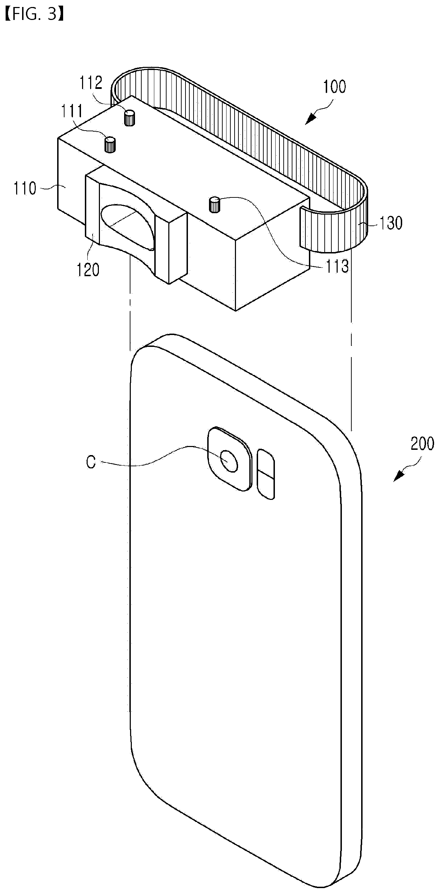

FIG. 3 is a perspective view illustrating a device according to the present invention.

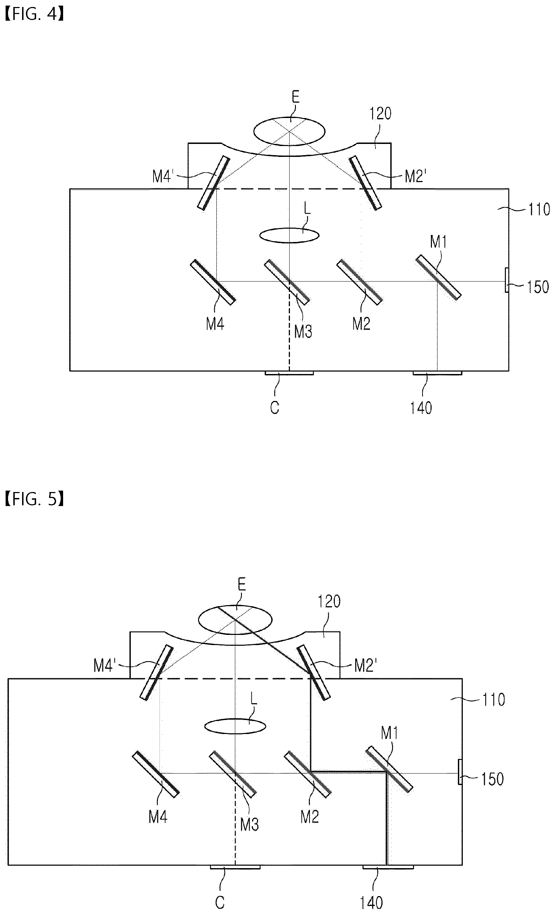

FIG. 4 is a longitudinal sectional view illustrating the device according to the present invention, which shows a view of optical paths.
FIGS. 5 to 7 are views for optical paths for describing operation methods of the device according to the present invention.

### [Modes of the Invention]

Hereinafter, a portable slit lamp device according to the present invention will be described with reference to FIGS. 3 and 4.

As illustrated in FIG. 3, a portable slit lamp device 100 according to the present invention may be installed on an image capture device 200. The image capture device 200 may be any device without limitation as long as the device is portable, is relatively small, and includes a camera C, and may be, for example, a smartphone.

The portable slit lamp device 100 includes a housing 110, an eye contact part 120 provided on the housing 110 toward an examinee's eye E, and a coupling part 130 provided on the housing 110 toward the image capture device 200.

In the description with reference to FIG. 3, the eye contact part 120 is positioned on a front surface of the portable slit lamp device 100, and examinee's right or left eye is positioned at the eye contact part 120. To this end, the eye contact part 120 may be formed of an elastic material.

Meanwhile, in one embodiment of the present invention, an auxiliary light source may be positioned in the eye contact part 120. The auxiliary light source prevents brightness of a captured image result of the camera C from being excessively lowered when the eye E of an examinee is pressed against the eye contact part 120 and surrounding light does not enter.

The coupling part 130 is positioned on a rear surface of the portable slit lamp device 100. The coupling part 130 may be formed of an elastic material and have a band shape to correspond to various types of image capture devices 200 and various positions of cameras C of the image capture devices, but the present invention is not limited thereto. For example, a user forcibly stretches the coupling part 130 of the portable slit lamp device 100, and inserts the image capture device 200 into the coupling part 130, and adjusts a position of the eye contact part 120 so that the camera C of the image capture device 200 is coaxial with a center of the eye contact part 120, and then, preparation for use is completed.

In the housing 110, a light source, a plurality of mirrors, and a lens are positioned so that the slit lamp emits light in a desired direction.

The portable slit lamp device 100 according to the present invention does not use a light source provided in the image capture device 200 and use a separate light source unlike the conventional technologies. This is to extract a precise image capturing result at maximum in consideration of both of a fact that a distance and a direction between the camera C and the light source vary for each image capture device and a fact that a quantity of light and a light emitting region of a light source vary.

A visible light source 140 which emits visible light is positioned in the housing 110, and an additional light source 150 which emits light with a different wavelength from visible light may be further positioned in the housing 110. For example, the light with the different wavelength may be infrared light.

The visible light source 140 and the additional light source 150 are provided on different surfaces in the housing 110, a visible light ray and a light ray with a different wavelength, which are emitted from the visible light source 140 and the additional light source 150, come into contact with each other at a predetermined portion, and a mirror M1 is positioned at the contact portion. The visible light ray and the light ray with the different wavelength are selectively totally reflected by or pass through the mirror M1, and the light rays introduced from light sources at different positions pass the corresponding mirror M1 and become the same light so that an optical path can be simplified and the number of components can be reduced.

A slit module (not shown) may be provided in the visible light source 140. In this case, vertically extending slit visible light is emitted from the visible light source 140 passes various mirrors, which will be described below, and arrives at the examinee's eye E.

In another embodiment, a visible light source 140 is a general light source (for example, light emitting diode (LED)), and slit modules (not shown) may be provided in a right reflecting mirror M2' and a left reflecting mirror M4' which will be described below. In this case, general visible light arrives at the right reflecting mirror M2' or the left reflecting mirror M4' through various mirrors, is reflected by the reflecting mirrors, and passes through the slit module (not shown) so that vertically extending slit visible light is generated and arrives at an examinee's eye E.

Since a technology of the slit module (not shown) is a widely known conventional technology, a detailed description thereof will be omitted.

In order for the mirror M1 to perform selective total reflection and transmission, the visible light source 140 and the additional light source 150 may be disposed so that an angle between the visible light source 140 and the additional light source 150 is a right angle. In an example illustrated in FIG. 4, the visible light source 140 is positioned on a surface in contact with the camera C of the image capture device 200, and the additional light source 150 is positioned on a right surface of the housing 110 so that an angle between the visible light source 140 and the additional light source 150 is a right angel. In another embodiment, a visible light source 140 may be disposed on a surface on which an eye contact part 120 is positioned, and in this case, a position of a mirror M1 is adjusted so that a reflective surface is directed in an up right direction in the drawing.

In the example illustrated in FIG. 4, the mirror M1 totally reflects a visible light ray and allows a light ray with a different wavelength from visible light to pass through the mirror M1. Accordingly, a visible light ray emitted from the visible light source 140 is totally reflected toward a mirror M2, and a light ray with a different wavelength from visible light emitted from the additional light source 150 passes through the mirror M1 and travels toward the mirror M2 as the same light as the visible light ray.

The mirror M2 totally reflects the visible light ray and allows the light ray with the different wavelength. Accordingly, the light ray with the different wavelength emitted from the additional light source 150 continuously travels toward a mirror M3.

The mirror M2 totally reflects the visible light ray but may be separately manipulated through a mirror control unit 112 (see FIG. 3) for the visible light ray to bypass the mirror M2. For example, when the mirror control unit 112 is rotated counter-clockwise by 45°, the visible light ray does not come into contact with the mirror M2 and continuously travels toward the mirror M3. Through this method, the user may allow the visible light ray to be totally reflected by the mirror M2 or to pass the mirror M2 without change. This is to control a slit lamp visible light ray to be emitted from a right side or left side with respect to the examinee's eye E.

The visible light ray totally reflected by the mirror M2 is reflected by a right reflecting mirror M2' and emitted toward the examinee's eye E from the right side. In this case, since the right reflecting mirror M2' is rotated by a right reflecting mirror control unit 111 (see FIG. 3) so that an angle of the right reflecting mirror M2' may be controlled, the angle may be controlled within 45° with respect to an axis of the examinee's eye E. Through such a function, a cameraman may capture an image of the entire eye by controlling only the right reflecting mirror control unit 111 without moving the examinee's eye E.

The visible light ray passing the mirror M2 continuously travels and arrives at the mirror M3. The mirror M3 allows the visible light ray to pass the mirror M3 and totally reflects the light ray with the different wavelength. Accordingly, the visible light ray passes through the mirror M3 and arrives at a mirror M4.

The mirror M4 totally reflects the visible light ray. The visible light ray totally reflected by the mirror M4 is reflected by a left reflecting mirror M4' and emitted toward the examinee's eye E from the left side. Similarly, the left reflecting mirror M4' is rotated by a left reflecting mirror control unit 113 (see FIG. 3) so that an angle of the left reflecting mirror M4' may be controlled.

The light ray with the different wavelength passing through the mirror M2 arrives at the mirror M3. The mirror M3 is positioned on an axis which is collinear with a central axis of the eye contact part 120 and an axis of the camera C of the image capture device 200. The light ray with the different wavelength is totally reflected by the mirror M3 and emitted to the examinee's eye E. This is because the light ray with the different wavelength should be straightly emitted to the examinee's eye E unlike the visible light ray emitted by the slit lamp.

Meanwhile, both of an image captured using the visible light ray reflected by the right reflecting mirror M2' or the left reflecting mirror M4' and an image captured using the light ray with the different wavelength reflected by the mirror M3 become clearer while passing through a lens L, pass through the mirror M3, and are captured by the camera C. For example, when the image capture device 200 is a smartphone, the images captured by the camera C may be stored in a memory and transmitted to a medical professional by using a communication function of the smartphone.

A case in which visible light is emitted to a right side of the examinee's eye E will be described with reference to FIG. 5.

The portable slit lamp device 100 is coupled to the image capture device 200, axes thereof are aligned, and when power is applied to the visible light source 140, a visible light ray is emitted in the form of a slit lamp visible light.

The emitted visible light ray is totally reflected by the mirror M1 and the mirror M2 and arrives at the right reflecting mirror M2'. The visible light ray reflected by the right reflecting mirror M2' is emitted to the examinee's eye E from the right side. In this case, the user may manipulate the right reflecting mirror control unit 111 to change an emission angle of the right reflecting mirror M2'.

An image of the examinee's eye E, to which the visible light ray in the form of the slit lamp visible light is obliquely emitted, passes through the lens L and the mirror M3, and is checked through the camera C.

In another embodiment of the present invention, a visible light source 140 emits general visible light, the visible light reflected by a right reflecting mirror M2' may be changed into the form of slit lamp visible light.

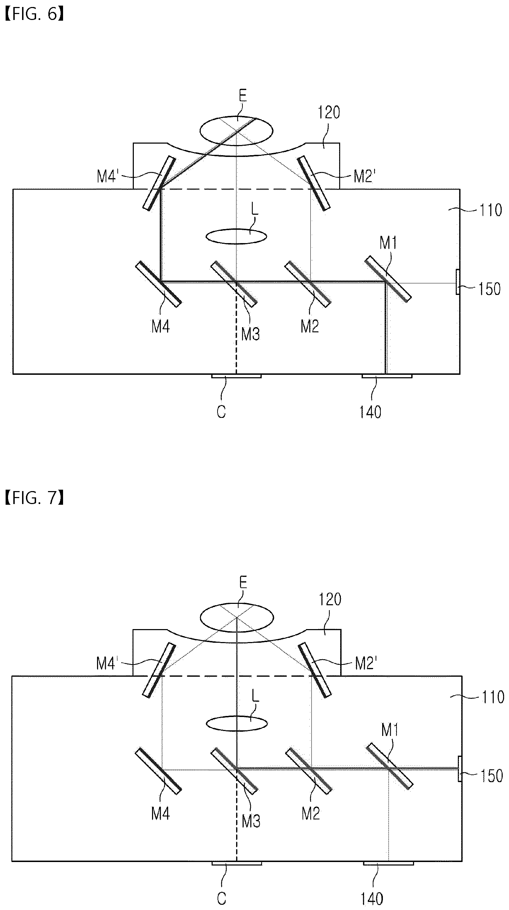

A case in which visible light is emitted to a left side of the examinee's eye E will be described with reference to FIG. 6.

The portable slit lamp device 100 is coupled to the image capture device 200, the axes thereof are aligned, the user manipulates the mirror control unit 112 for a visible light ray does not arrive at the mirror M2. Then, when power is applied to the visible light source 140, a visible light ray is emitted in the form of a slit lamp visible light.

The emitted visible light ray is totally reflected by the mirror M1 passes through the mirror M2 without change, passes through mirror M3, is totally reflected again by the mirror M4, and arrives at the left reflecting mirror M4'. The visible light ray reflected by the left reflecting mirror M4' is emitted to the examinee's eye E from the left side. In this case, the user may manipulate the left reflecting mirror control unit 113 to change an emission angle.

An image of the examinee's eye E to which the visible light ray in the form of the slit visible lamp light is obliquely emitted passes through the lens L and the mirror M3 and is checked through the camera C.

Similarly, visible light reflected by the left reflecting mirror M4' may be changed into the form of slit lamp visible light.

A case in which light with a different wavelength is emitted to the examinee's eye E will be described with reference to FIG. 7.

The portable slit lamp device 100 is coupled to the image capture device 200, the axes thereof are aligned, and when power is applied to the additional light source 150, the light ray with the different wavelength is emitted.

The light ray with the different wavelength passes through the mirror M1 and the mirror M2, is totally reflected by the mirror M3, and is directly emitted to the examinee's eye E.

An image of the examinee's eye E to which the light ray with the different wavelength is emitted passes through the lens L and the mirror M3 and is checked through the camera C.

The present invention has been described with reference to embodiments illustrated in the accompanying drawings so that those skilled in the art can easily understand and reproduce the present invention, but this is merely exemplary. It will be understood by those skilled in the art that various modifications and equivalent other example embodiments may be made from the embodiments of the present invention. Therefore, the scope of the present invention is defined by the appended claims.

### [Reference Numerals]

100: PORTABLE SLIT LAMP DEVICE
110: HOUSING
111: RIGHT REFLECTING MIRROR CONTROL UNIT
112: MIRROR CONTROL UNIT
113: LEFT REFLECTING MIRROR CONTROL UNIT
120: EYE CONTACT PART
130: COUPLING PART
140: VISIBLE LIGHT SOURCE
150: ADDITIONAL LIGHT SOURCE
C: CAMERA
M1, M2, M3, M4: MIRROR
M2': RIGHT REFLECTING MIRROR
M4': LEFT REFLECTING MIRROR

## Claims

1. A portable slit lamp device (100) which is installed on an image capture device (200) and used, the portable slit lamp device (100) comprising:
a housing (110) provided with a plurality of mirrors;
an eye contact part (120) provided on the housing (110) toward an examinee's eye; and
a coupling part (130) provided on the housing (110) toward the image capture device (200),
wherein the portable slit lamp device (100) and the image capture device (200) are coupled by the coupling part (130) so that a central axis of the eye contact part (120) is collinear with an axis of a camera (C) of the image capture device (200),
a visible light source (140) is positioned in the housing (110), and
by manipulating a mirror (M2), visible light emitted by the visible light source (140) is totally reflected by the mirror (M2), is reflected by a right reflecting mirror (M2'), and is emitted from a right side of an examinee's eye as slit visible light, or the visible light emitted by the visible light source (140) passes the mirror (M2), is reflected by a left reflecting mirror (M4'), and is emitted from a left side of the examinee's eye as slit visible light.

2. The portable slit lamp device of claim 1, wherein:
an additional light source (150), which emits light with a different wavelength from visible light, is positioned in the housing (110); and
the mirror (M2) totally reflects the visible light and allows the additional light to pass through the mirror (M2).

3. The portable slit lamp device of claim 2, wherein:
the visible light source (140) and the additional light source (150) are provided on different surfaces in the housing (110);
a mirror (M1) is positioned at a portion at which a visible light emitted by the visible light source (140) and an additional light emitted by the additional light source (150) come into contact with each other;
the mirror (M1) totally reflects the visible light and allows the additional light to pass through the mirror (M1); and
the visible light and the additional light are the same at downstream from the mirror (M1).

4. The portable slit lamp device of claim 3, wherein:
the mirror (M2) is positioned on a path of the same ray at downstream from the mirror (M1);
a mirror (M3) is positioned at downstream from the mirror (M2);
the mirror (M3) allows the visible light to pass through the mirror (M3) and totally reflects the additional light; and
the mirror (M3) is positioned on a line extending from the central axis of the eye contact part (120) that is collinear with the axis of the camera (C) of the image capture device (200).

5. The portable slit lamp device of claim 4, wherein:
the right reflecting mirror (M2') is positioned on a path of the visible light totally reflected by the mirror (M2);
a mirror (M4) is positioned on a path of the visible light passing through the mirror (M3);
the mirror (M4) totally reflects the visible light; and
the left reflecting mirror (M4') is positioned on a path of the totally reflected visible light.

6. The portable slit lamp device of claim 5, wherein the right reflecting mirror (M2') and the left reflecting mirror (M4') are rotatable.
